# EUROPÄISCHE PATENTANMELDUNG

(11) **EP 0 778 081 A1**
(43) Veröffentlichungstag der Anmeldung: **11.06.1997**
(21) Anmeldenummer: 96118817.4
(22) Anmeldetag: 25.11.1996
(51) Int. Cl.: B01F 15/00, C07C 63/68, C07C 63/70

(54) **Verfahren zur Herstellung von fluorhaltigen chemischen Verbindungen**

(30) Priorität: 01.12.1995 DE 19544871
(71) Anmelder: HOECHST AKTIENGESELLSCHAFT, 65929 Frankfurt am Main (DE)
(72) Erfinder: Pfirmann, Ralf, Dr., 64347 Griesheim (DE); Seitz, Friedrich Dl., 65760 Eschborn (DE)

(57) **Zusammenfassung**

Verfahren zur Herstellung fluorhaltiger Verbindungen durch Reduktion in einem mit einem Rührer versehenen Behälter, wobei der Rührer ganz oder teilweise aus einem Kohlenstoffmaterial aufgebaut ist.

## Beschreibung

Verfahren zur Herstellung von fluorhaltigen Verbindungen zeichnen sich dadurch aus, daß, besonders in sauren Medien, zum Teil eine sehr starke Korrosion durch Fluorwasserstoff auftritt. Dadurch wird die weitere Korrosion verstärkt, die durch andere Einflüsse verursacht wird. Zudem wird durch die Anwesenheit von Fluoridionen die Passivschicht an Metallen zerstört, sodaß dadurch auch eine Korrosion durch andere Mischungsbestandteile erleichtert wird. Dies verhindert nicht nur den Einsatz von Edelstahlgarnituren, sondern auch von Emailgarnituren, da Email wegen der Bildung von Hexafluorkieselsäure starken Abtrag erfährt, und die Abtragsgeschwindigkeit bei beginnender Korrosion progressiv stark steigt.

In der Regel weicht man bei niedrigen Temperaturen, d.h. bis etwa 60°C, auf Polyethylen (PE)-Apparaturen oder entsprechend ausgekleidete Apparate aus. Bis zu Temperaturen von 100°C bis etwa 160°C ist auch die Verwendung von Polytetrafluorethylen (PTFE) möglich, jedoch schon mit erheblichen Problemen verbunden. So müssen bei Temperaturen über 120°C Sonderkonstruktionen, insbesondere im Wärmeaustausch- und Rührwerksbereich, verwendet werden. In kalten Apparateilen können auch Teile auch Polyvinylidenfluorid (PVDF) verwendet werden, das zwar besser als PTFE verarbeitbar ist, aber eine deutlich geringere Erweichungstemperatur besitzt und daher in seiner Anwendungsbreite, besonders für mechanisch stark belastete Teile wie Rühreinrichtungen, stark beschränkt ist. Insbesondere hohe Drehmomente, die beim Anfahren der Rührwerke auftreten, führen aufgrund mangelnder Verwindungssteifigkeit zu Verformungen.

Andere Fluorkunstoffe (z.B. Fluoroshield®) sind ebenfalls prinzipiell gegen saure, fluorwasserstoffhaltige Medien stabil. Diese Kunstoffe (PTFE, PVDF, Fluoroshield) haben jedoch unter anderem folgende Nachteile. Zum einen sind die Wärmedurchgangsskoeffizienten dieser Kunststoffe so gering im Vergleich zu herkömmlichen Materialien wie z.B. Metallen, daß man gezwungen ist, die Wandstärken gering zu halten. Aufgrund der mechanischen Eigenschaften dieser Kunststoffe (Fließverhalten bei Annäherung an den Erweichungspunkt, Druck-, Zug- und Scherfestigkeit, Verwindungssteifigkeit) können die Apparate nicht aus massiven Material hergestellt werden, sondern die Kunststoffe können nur zur Auskleidung verwendet werden oder müssen aufgesintert werden. Zum anderen sind die Materialien wegen der lipophilen Eigenschaften und ihrer in der Regel geringen Dichte gegen die Diffusion von organischen Verbindungen und vor allem gegen die Diffusion von Fluorwasserstoff nicht stabil. Dies führt aber dazu, besonders bei Umsetzungen unter Druck oder bei höherer Temperatur und erleichtert durch die aufgrund der geringen Wärmedurchgangskoeffizienten und technischen Gegebenheiten geringen Wandstärken, daß in der Regel nach der Inbetriebnahme der Apparate schnell eine Korrosion durch Fluorwasserstoff bzw. Flußsäure (insbesondere lokale Korrosionsphänomene) an den metallischen Außenbehältern auftritt. Trotz der in der Regel geringen Wandstärken stellen die kunststoff-beschichteten Apparate nur ein unbefriedigende Lösung dar, da die Wärmedurchgangskoeffizienten so gering sind, daß dennoch kein exothermen oder endothermen Umsetzungen durchgeführt werden können, da Erhitzen oder Kühlen nur schlecht gelingt. Weitere Nachteile der Fluorpolymer-Werkstoffe sind die schlechte Verarbeitbarkeit und die Beschränkung auf Verfahren unter Normal- und unter Überdruck, da die Anwendung von Unterdruck kaum möglich ist, weil sich die Schichten wegen ihrer geringen Haftung von den Außenbehältern ablösen und unregelmäßig verformen.

Unter den häufig vorkommenden Umsetzungsbedingungen, besonders in wäßrigen Lösungen, befindet sich stets Flußsäure im Dampfraum oder es muß sogar säurehaltiger Wasserdampf beim Entspannen von Reaktionsgas kondensiert werden. Korrosion durch kondensierende verdünnte Flußsäure kann aber mit herkömmlichen Methoden kaum beherrscht werden.

Es besteht also ein Bedürfnis nach einem einfachen und universell anwendbaren Verfahren, das neben herkömmlichen Reaktionen auch Umsetzungen erlaubt, die Fluorwasserstoff in der Reaktionsmischung enthalten, insbesondere in wäßrigen Lösungen.

Nach Römpp, Chemie-Lexikon, 5. Auflage 1962 (Bd. 1, S. 1125) ist Diabon® ein säureresistenter Werkstoffe aus porösem Graphit, der durch Imprägnierung mit Harzen flüssigkeitsdicht gemacht wurde. Dieser ist gegen Fluorwasserstoff und andere Mineralsäuren beständig.

Hinweise, daß die Verwendung von Behältern aus kohlenstoffhaltigen Material Vorteile bietet, da mechanische Festigkeit, gute Wärmedurchgangseigenschaften und hohe Korrosionsbeständigkeit zusammenkommen, finden sich auch in der folgenden Literatur:
J. Künzel, Chem.-Tech. (Heidelberg), 17 (12), 16, 18, 20 (1988);
J. Künzel in VDI-Berichte 674, 87-103 (1988);
H. Böder, E. von Gellhorn, J. Künzel, Chem.-Ing.-Tech. (1987) 59 (2), 122-126;
J. Künzel, A. Swozil, H. Würmseher, Swiss Chem. (1983) 5 (10a), 17/22;
G.L. Hart, G. Pritchard, in Carbon Fibres, 2nd int. conf. Proc. London, Feb 18-20, 1974, paper 34;
J. Künzel, E. von Gellhorn, H. Böder, in Composite Polymers, 1 (6) (1988),
K.S. Lally, W.C. Webster, R.N. Salzman, Chem. Engng. Prog. 84 (11) (1988), Chem. Engng. 93 (10) (1986), 47.

Gegenstand der Erfindung ist ein Verfahren zur Herstellung fluorhaltiger Verbindungen durch Reaktion in einem mit einem Rührer versehenen Behälter, dadurch gekennzeichnet, daß man mit einem Rührer arbeitet, der ganz oder teilweise aus einem Kohlenstoffmaterial aufgebaut ist.

Die Rührorgane des bei dem erfindunsgemäßen Verfahren benutzten Rührers bestehen aus kohlenstoffhaltigem Material. Die Rührwelle des Rührers dagegen kann auch aus Metall bestehen, es ist aber auch möglich, daß diese Welle ebenfalls aus kohlenstoffhaltigem Material besteht. Dieses Material kann mit Phenol-, Expoxid-, Polyimid-, oder Polyesterharzen oder auch mit Fluorkunststoffen gefüllt, d.h. gepreßt, gesintert oder auf andere Weise zu einen festen Werkstück geformt sein. Als Füllstoffe für das Kohlenstoff-Material werden in der Regel Furan- oder Phenolharze oder Fluorkunststoffe verwendet, zum Beispiel CTFE/VDF (Poly(chlortrifluorethylen-co-vinylidenfluorid)), PTFE (Polytetrafluorethylen), ECTFE (Poly(ethylen-co-chlortrifluorethylen)), ETFE (Poly(ethylen-co-tetrafluorethylen)), FEP (Poly(tetrafluorethylen-co-hexafluorpropylen)), PCTFE (Polychlortrifluorethylen), PVDF (Polyvinylidenfluorid), PVF (Polyvinylfluorid), TFB (Polytetrafluorethylen-co-hexafluorpropylen-co-vinvlidenfluorid]) oder CM-X (Poly(hexafluorisobutylen-co-vinylidenfluorid)). Weitere mögliche Fluorkunststoffe sind zum Beispiel beliebige Copolymere von Hexafluorisobutylen, Hexafluorpropylen, Tetrafluorethylen, Vinylfluorid und Vinylidenfluorid bzw. analoger Verbindungen. Diese Fluorpolymere können außerdem überall dort eingesetzt werden, wo PTFE oder PVDF angegeben sind.

Die Rührwelle des Rührers kann bevorzugt aus mit Kohlenstoff-Fasern verstärktem Kunststoff (CFK) oder aus mit Kohlenstoff-Fasern verstärktem Kohlenstoff (CFC) bestehen. Die Kohlenstofffasern in diesem Material haben im allgemeinen einen Durchmesser von etwa 1 bis etwa 100 µm, bevorzugt von etwa 3 bis 30 µm, insbesondere von 5 bis 15 µm, wobei der Fasergehalt bei CFK-Werkstoffen etwa 30 bis 90 %, vorzugsweise 50 bis 70 % beträgt. Bei den Fasern handelt es sich vorzugsweise um hochmodule oder hochfeste Fasern, wobei die Fasern gegeneinander Winkel von 0 bis 45° einnehmen können. Ebenso können Faserverbundwerkstoffe zum Einsatz kommen. Die Kohlenstoffasern können auch in Form entsprechender Filamente vorliegen. Die CFK and CFC-Materialien haben im allgemeinen Biegefestigkeiten von etwa 50 bis 2000 N/mm², vorzugsweise 80 bis 1500 N/mm², insbesondere 100 bis 800. Die Werte für den E-Modul (Zug) betragen etwa 10 000 bis 400 000 N/mm², vorzugsweise etwa 25 000 bis 130 000 N/mm², die Zugfestigkeit beträgt etwa 0,05 bis 8, vorzugweise etwa 0,3 bis 1 N/mm². Die Dichte diesen Materialien beträgt etwa 1,2 bis 2,0, vorzugsweise 1,55 bis 1,6 g/cm³.

Als Beispiele von Fasern bzw. Fasergelegen zur Verstärkung von Kunststoffen können die Produkte Sigrafil C40, SFC 6 oder 12 oder Sigratex (verschiedene Typen) oder 320 angeführt werden. Die entsprechenden Stoff- und technischen Daten können z.B. den technischen Informationsblättern der SGL Carbon entnommen werden.

Ebenfalls mögliche Konstruktionswerkstoffe sind die SIGRABOND®-Typen der SGL Carbon, die sowohl als CFC (Kohlenstoffaser-verstärkter Kohlenstoff) als auch als CFK (kohlenstoffverstärkter Kunststoff) zur Verfügung stehen. Diese können auch mit keramischen Materialien wie z.B. SiC beschichtet werden. So sind die Typen CC 1001 G und CC 1501 G (jeweils CFC) jeweils unbeschichtet und mit Silicum-infiltrierten Siliciumcarbid oder α-SiC beschichtet sowie als CFT CC 1506 G geprüft worden. Technische Daten können den entsprechenden Informationsblättern entnommen werden.

Die in Frage kommenden CFK-Materialien können die weiter unten beschriebenen Kitte enthalten oder können mit Epoxid-, Phenol-, Furanharzen oder mit Fluorkunstoffen wie zum Beispiel Polytetrafluorethylen (PTFE) gefüllt sein. Die Polymermatrix bei solchen CFK-Materialien kann aus ungesättigten Polyestern, Phenolharzen, Epoxidharzen oder Polyimidharzen bestehen, bevorzugt sind Duroplasten.

Die Welle der Rührerkonstruktion (Kohlenstoffmaterial) besteht in der Regel aus massivem Material oder aus einer Hohlrohrkonstruktion, die gegebenenfalls mit einem Kern aus mechanisch festem Material gefüllt sein kann, z.B. können Metalle wie die Werkstoffe 1.4571 und 2.4610, massiv oder als Hohlrohr als Kern der Welle eingebaut werden. Solche Hohlkonstruktionen können, sofern gewünscht, zur Verhinderung von Korrosion auf der Innen- und Außenseite mit den nachfolgend beschriebenen Kittmaterialien abgedichtet werden. Da diese Konstruktionen in der Regel an eine den Rührer betreibende, aus Metall bestehende Konstruktion, in der Regel Werkstoff der Nr. 1.4571, angefügt sind, ist diese Übergangstelle mittels der beschriebenen Möglichkeiten besonders abzudichten.

Die Rührgeometrie des verwendeten Rührers kann gemäß allen technisch bekannten Varianten ausgeführt werden (sh. u.a. M. Zlokarnik, H. Judat in Ullmann's Enzyklopedia of Industrial Chemistry, Vol. B2 (1992), Kapitel 25). So sind ein- und mehrstufige Rührer möglich. Anwendbar sind Blattrührer, Kreuzbalkenrührer, Ankerrührer, Impeller- oder Propellerrüher, Turbinen- und Ankerrührer oder auch spezielle Ausführungsformen dieser Typen wie zum Beispiele MIG® oder INTERMIG®, die eine besonders intensive Durchmischung bei gleichzeitiger Optimierung der Energieaufnahme gewährleisten (bevorzugt sind Blatt-, Kreuzbalken- und Ankerrührer). Für spezielle Probleme, z.B. sehr zähe Reaktionsmischungen sind die in der angegebenen Literatur aufgeführten technischen Lösungen ebenso möglich. Die Größe und Form der Rührorgane kann den jeweiligen Erfordernissen des Reaktionsapparates und der Umsetzung angepaßt werden.

Die Länge der Rührwelle ist abhängig von der Größe des Reaktors und beträgt im allgemeinen 50 bis 95 %, insbesondere 70 bis 90 %, der Reaktorhöhe. Der Durchmesser der Rührwelle beträgt ungefähr 1 bis 100 cm, wobei im einzelnen folgende Werte üblich sind: 1 bis 4 cm bei Laboranlagen, 5 bis 30 cm bei Pilotanlagen, 40 bis 100 cm bei Produktionsanlagen. Das jeweilige Material der Rührwelle ist davon abhängig, ob die Rührwelle hohl oder massiv ausgestaltet ist. Bei Hohlwellen ist die Materialdicke 2 bis 20 %, bevorzugt 5 bis 15 %, bezogen auf den Gesamtdurchmesser der Welle. Gleiches gilt für die Rührorgane.

Die radialen Abmessungen der Rührorgane sind abhängig von dem Behälter, in dem der Rührer eingesetzt werden soll. Üblicherweise sind die Rührorgane so ausgestaltet, daß sie 10 bis 90, vorzugsweise 30 bis 80 % des Behälterdurchmessers ausmachen. Die Höhe der Rührerorgane ist stark abhängig vom Rührertyp und beträgt etwa 3 bis 20 % der Höhe des Behälters.

Erfindungsgemäß bevorzugt wird eine durch eine Gleitringdichtung, Magnetkupplung oder Stopfbüchsabdichtung gegen außen abgedichtete Rühreinrichtung verwendet. Diese Bauteile werden in der konstruktiv üblichen Art verwendet. Wesentlicher Bestandteil einer solchen Abdichtung über Gleitringe ist die Verwendung von keramischen, korrosionsbeständigen Gleitringmaterialien, wie zum Beispiel Bornitrid, Borcarbid, Siliciumnitrid oder Siliciumcarbid, da diese die geforderte Härte und somit Abriebfestigkeit aufweisen. Diese Materialien können in verschiedenen Varianten eingesetzt werden, so zum Beispiel durch Variation des Herstellungsverfahrens (unterschiedliche Sinterung, Korngröße) oder durch Variation der Bestandteile des Materials (z.B. nicht stöchiometrische Anteile der Komponente im Material wie in Silicium-infiltriertem Siliciumcarbid), was eine Anpassung der tatsächlichen Materialeigenschaften an die chemischen und mechanischen Erfordernisse erlaubt.

Durch die Konstruktionsweise weist der erfindungsgemäße Rührer Vorteile hinsichtlich der Materialfestigkeit auf, verglichen mit anderen korrosionsbeständigen Materialien, wie z.B. Fluorkunststoffen.

Bei dem erfindungsgemäßen Verfahren kann nicht nur der Rührer allein, sondern zusätzlich auch der Behälter für die Reaktion aus dem kohlenstoffhaltigen Material bestehen oder mit diesem Material ausgekleidet sein. Dies bedeutet, daß entweder die Bauteile für den Behälter in massiver Form aus diesem Material bestehen können oder daß lediglich die Oberfläche mit diesen Materialien überzogen ist. Wesentlich ist hierbei, daß diejenigen Teile des Behälters, die mit dem Produkt in Berührung kommen, aus den kohlenstoffhaltigen Materialien bestehen oder damit überzogen sind.

Wie literaturbekannt ist, weist durch spezielle Verfahren wie zum Beispiel Carbonisierung erzeugter Graphit extrem hohe Druck- und Zugmodule auf. Zudem ist das Material weitgehend diffusionsstabil, besonders, da wie oben erläutert, entsprechende Wandstärken keinerlei Probleme darstellen. Die in der erfindungsgemäßen Verwendung vorteilhaften Behälter-Wandstärken können zwischen etwa 5 und etwa 200 mm liegen, insbesondere zwischen etwa 20 und etwa 80 mm.

Zum Abdichten und Verfugen sowie zum Schutz nicht inerter Bauteile bei Rührern und Behältern nimmt man hierfür übliche mit organischen Harze wie zum Beispiel Epoxid-, Melamin-, Furan-, Alkyd-, Vinyl-, Polyester-, Urethan- oder Phenolharzen gefüllte Materialien. Speziell sind hier Asplit®-Typen sinnvoll, insbesondere die Typen CV, CL, CN, ET, OC, OQ, FN und Säurekitte HB, HES HB, HFR bzw. Feuerkitte K12, K14, K16. Genauso verwendbar sind aber auch analoge Typen dieser Harze, die unter anderen Bezeichnungen gehandelt werden. Erläuterungen zur genauen Zusammensetzung solcher Kitte, insbesondere von Kitten mit organischen Bindern, finden sich in Ullmann' Encyclopedia of Industrial Chemistry. Bd. 5A 5th, (1986), S. 539 bis 544. Bevorzugt sind die handelsüblichen Kitte Asplit CN, ein Kitt vom Typ Phenol-Formaldehyd-Harze und Asplit FN, ein Kitt vom Typ Furanharz, Abwandlung zu anderen Typen sind jedoch je nach Art des Reaktionsmediums sinnvoll. Dies kann gut am Beispiel dieser beiden Asplit-Typen erläutert werden, Typ CN hat nämlich Vorteile im sauren Bereich, Asplit FN im alkalischen Medium, von der Lösemittelbeständigkeit sind beide Typen in etwa äquivalent.

Daneben ist es auch möglich, Dichtstellen oder nicht per se inerte Bauteile durch Schichten aus inertem Material, insbesondere durch Edelmetalle wie Gold oder Platin zu schützen. Ebenfalls ist es möglich, solche besonders gefährdeten Teile massiv aus Edelmetallen zu fertigen. Die Metallschichten können aufplattiert, aufgeschmolzen, gesintert oder gedampft werden.

Insbesondere kann das erfindungsgemäße Verfahren zur Herstellung organischer Fluorverbindungen eingesetzt werden. Hierunter fallen sowohl aliphatische als auch aromatische Fluor-Verbindungen, wie zum Beispiel Tri- oder Tetrafluorpropan-carbonsäure, 2,3,4,5-Tetrafluorbenzoesäure, 5-Fluor-2-nitrophenol oder ähnliche Verbindungen. Besonders bevorzugt sind Synthese- oder Aufarbeitungschritte von fluorhaltigen Verbindungen, bei denen diese Verbindungen, besonders bei erhöhten Temperaturen, in sauren, besonders in stark sauren Lösungen, gehandhabt werden. Bei diesen Verfahrensschritten zeigt es sich, daß stets geringe Mengen Fluorid aus dem organischen Material abgespalten werden, die zur Korrosion Anlaß geben. Nützlich ist das erfindungsgemäße Verfahren auch bei Verbindungen, die neben Fluor noch Chlor enthalten und wo analog Chlorid-Ionen bzw. HCl freigesetzt wird, die ebenfalls korrosiv wirken. Die Konzentration an Halogenid insgesamt in dem Reaktionsansatz kann 5 ppm bis 25 % betragen.

Bevorzugt wid das erfindungsgemäße Verfahren auf fluoraromatische Verbindungen als Reaktanden oder als Produkte angewandt, insbesondere auf fluoraromatische Carbonsäuren und/oder Phenole, Alkohole oder deren funktionelle Derivate wie Ester, Amide, Halogenide, Aldehyde, Benzylalkohole, Ether, Benzylhalogenide, bevorzugt Benzylfluoride, Benzalhalogenide, bevorzugt Benzalfluoride, Benzotrihalogenide, bevorzugt Benzotrifluoride, die darüberhinaus sauer oder basisch reagierende Gruppen enthalten. Im einzelnen läßt sich das erfindungsgemäße Verfahren zum Beispiel zur Herstellung folgender Verbindungen nutzen:
Tetrafluorphthalsäure, 2,3,4,5-Tetrafluorbenzoesäure, 3-Hydroxy-2,4,5-trifluorbenzoesäure, 4-Hydroxy-2,3,5-trifluorbenzoesäure, 4-Amino-3,5,6-trifluorphthalsäure, 2,3-Dichlor-4,5-difluorbenzoesäure, 3-Amino-2,4,5-trifluorphthalsäure, 4-Chlor-3,5,6-trifluorphthalsäure, 3-Chlor-2,4,5-trifluorbenzoesäure, 4-Hydroxy-3,5,6-trifluorphthalsäure, 3,5,6-Trifluorphthalsäure, 2,4,5-Trifluorbenzoesäure, 2-Chlor-4,5-difluorbenzoesäure, 2-Chlor-6-nitrophenol, 2-Chlor-3-fluor-6-nitrophenol, 5-Fluor-2-nitrophenol und 2,3-Difluor-6-nitrophenol.

Das erfindungsgemäße Verfahren umfaßt insbesondere folgende Reaktionstypen: Halogen-Alkoxid Austausch, Halogen-Hydroxid-Austausch, Halogen-Amin-Austausch (jeweils insbesondere Austausch von Fluoratomen), Halogen-Halogen-Austausch, Decarboxylierung, Decarbonylierung, Hydrolyse von Nitrilen, Amiden, Anhydriden, Estern, Säurechloriden, Imiden, Schiemann- und Balz-Schiemann-Reaktion, Bamberger-Umlagerung, Veretherung, Acylierung, insbesondere in flüssigem Fluorwasserstoff, der gegebenenfalls Wasser enthalten kann.

Die erfindungsgemäßen Verfahren können in wässriger Lösung und in Lösungen in den üblichen organischen Lösemitteln durchgeführt werden, wässrige Lösungen sind bevorzugt. Die Viskosität des Reaktionsgemisches beträgt ungefähr 0,1 bis 5 000 cP, vorzugsweise 1 bis 1 000 cP, insbesondere 25 bis 250 cP. Die Umdrehungszahl des Rührers liegt im allgemeinen bei 1 bis 2 000 Umdrehungen pro Minute, bei Laborverfahren bei 100 bis 1000 U/min, bei Pilotanlagen bei 5 bis 200 U/min und bei Produktionsanlagen bei 2 bis 100 U/min.

Das erfindungsgemäße Verfahren kann in Behältern von etwa 0,5 l (Labormaßstab) bis etwa 20 m³ durchgeführt werden, bevorzugt sind Anlagen mittlerer Größe, die Reaktionseinheiten von etwa 200 l bis etwa 5 m³ beinhalten.

Man kann erfindungsgemäß bei Temperaturen zwischen etwa -20° und etwa 220°C arbeiten, bevorzugt zwischen 50° und 180°C, insbesondere bei 100 bis 180°C. Die möglichen Drücke betragen 0,05 bis 4,0 MPa, vorzugsweise 0,1 bis 2,0 MPa.

Besonders vorteilhaft ist die Verwendung der erfindungsgemäß einzusetzenden Apparaturen, wenn ungereinigte Reaktionsgemische weiterverarbeitet werden sollen, da diese höhere Mengen an freiem Fluorid enthalten können. Insbesondere kann dies der Fall sein, wenn durch nucleophile Austauschreaktionen Funktionalisierungen an organischen, aliphatischen oder aromatischen Verbindungen vorgenommen werden, wenn diese Funktionalisierungen per se Fluoridionen freisetzen und die Mischungen zur Auf- oder Weiterverarbeitung angesäuert werden müssen, wie dies bei der Synthese von Phenolen oder speziellen Carbonsäuren vorkommt. Dadurch kann jeweils ein Reinigungsschritt eingespart werden, wodurch eventuelle Ausbeuteverluste verhindert und als auch Fertigungkosten eingespart werden.

Es versteht sich von selbst, daß das erfindungsgemäße Verfahren auf das Arbeiten mit nicht-oxidierenden Medien beschränkt ist, da das kohlenstoffhaltige Material durch Oxidationsmittel angegriffen wird. Dies bedeutet, daß besonders Reaktionen mit Schwefelsäure von über 70 % Gehalt oder mit Halogenen, wie Chlor oder Brom, oder mit Wasserstoffperoxid Beschränkungen unterliegen. Bei sehr niedrigen Temperaturen, d.h. im allgemeinen bei Temperaturen unter etwa 40°C, sind aber Ausnahmen durchaus möglich.

Es ist ein großer Vorteil des erfindungsgemäßen Verfahrens, daß die verwendeten Materialien, sofern sie nicht per se absolut inert bei den in Frage kommenden Umsetzungsbedingungen sind, so beschaffen sind, daß deren Abnutzung, Abtrag oder Oberflächenveränderung sich nicht beschleunigend auf eine weitere Korrosion auswirkt.

Damit stehen diese Materialien im wesentlichen Gegensatz zu metallischen Werkstoffen oder Email, die bei beginnendem Angriff stets progressive Korrosionsneigung zeigen, allein schon durch die Oberflächenvergrößerung.

Das erfindungsgemäße Verfahren unter Einsatz einer speziellen Rühreinrichtung hat auch den Vorteil, daß man auf den Zusatz von fluoridabfangenden Mitteln (s. R. Lorentz in "Werkstoffe und Korrosion" 9/83, Inhibition des Säureangriffs auf Chemieemail), die zudem in wäßrigen, sauren Lösungen in ihrer Wirksamkeit beschränkt sind, verzichten kann. Somit entfallen sowohl die Materialkosten für diese unproduktiven Zusätze als auch die in der Regel nicht zu vernachlässigenden Verfahrensprobleme, die sich besonders bei der Entfernung der anfallenden festen Niederschläge wie Siliciumdioxid oder Calciumfluorid ergeben, welche sehr schwer filtrierbar sind.

Die folgenden Beispiele erläutern das Verfahren, ohne es zu beschränken.

### Beispiel 1

### Herstellung von 2,3,4,5-Tetrafluorbenzoesäure

In 120 g Wasser und 6.0 g 96 %ige Schwefelsäure wurden 3.4 g Calciumhydroxid und 27.0 g rohes Oktafluorbisphthalimid (85.5 %ig) suspendiert.

Die Mischung wurde in einen 0.75 l Hastelloy® C4-Autoklav gegeben, in den ein Rührer eingebaut worden war. Der Rührer bestand aus einem 22 cm langen Hohlrohr (Außendurchmesser 2 cm), gefertigt aus Kohlenstoffaser-verstärktem Kohlenstoff (SIGRABON D von SGL Carbon). Das Rohr war mit 3 Einschnitten längs der Rührachse versehen (jeweils um 90° versetzt), in die je 1 Rührerblatt aus demselben Material (Größe 3 cm hoch, 6 cm breit), rechtwinklig eingeschoben und mit PTFE-Band befestigt wurde (Kreuzbalkenrührer). Die Rührerwelle wurde mit Stiften aus Hastelloy C4 an den Antrieb (Edelstahl 1.4571) angebracht und mit Asplit FN abgedichtet. Der Rührer wurde mit einer Umdrehunggeschwindigkeit von 200 U/min betrieben. Man erhitzte die Mischung 12 h in einem PTFE-Autoklaven auf 160°C unter Anwesenheit einer Email-Werkstoffprobe, die am Rührer mittels PTFE-Band befestigt wurde. Nach Beendigung der Reaktionszeit war der Email-Belag der Probe vollständig abgetragen, die Probe also vollständig zerstört.
Die Umsetzung erwies sich nach GC-Kontrolle als vollständig, die Mischung wurde über kalibrierte GC analysiert. Es wurden 17.7 g (91 mMol, 86.2 %) 2,3,4,5-Tetrafluorbenzoesäure nachgewiesen, die direkt weiterverarbeitet wurde.

Unter analogen Bedingungen setzte man eine Probe aus V4A-Stahl (Werkstoff-Nr. 1.4571) ein, wobei die Probe sich etwa je zur Hälfte im Gas- und Flüssigkeitsraum befand. Nach Ende der Umsetzung stellt man fest, daß der untere Teil der Probe nahezu vollständig fehlte, der im Gasraum eingesetzte Teil starken Abtrag, Loch- und Spannungsrißkorrosion aufwies.

Wurden unter denselben Bedingungen Graphitblöcke der Materialien Diabon von SGL Carbon, gefüllt mit Phenol-Formaldehyd-Harz, NS 2, feingekörnte Variante, NS 1 und NS 2 als potentielles Behältermaterial während 200 Stunden eingesetzt, so konnte kein chemischer Angriff und auch kein Verlust der mechanischen Festigkeit festgestellt werden.
Der Rührer zeigte ebenso wie die getesteten Kohlenstoffteile keinerlei Veränderungen unter diesen Bedingungen, insbesondere auch nicht die Rührerblätter der Kreuzbalkenrührer-Konstruktion.

### Beispiel 2

### Herstellung von 5-Fluor-2-nitrophenol

In diesem Beispiel wurde der in Beispiel 1 beschriebene Rührer über eine Gleitringdichtung in einen ansonsten handelsüblichen PTFE-Reaktor eingebracht, wobei die Gleitringdichtung aus α-Siliciumcarbid gefertigt war. Statt Asplit FN wurde Asplit CN und als Wellenmaterial CFK (kohlenstoffverstärkter Kunststoff auf Basis Phenolharz) eingesetzt. Im PTFE-Reaktor wurden 119.4 (0.75 Mol) 2,4-Difluornitrobenzol vorgelegt, bei 55°C in 4 h 104,8 g (1.566 Mol) Kaliumhydroxid (85 %ig) in 300 g Wasser zugetropft, danach wurde die Temperatur auf 60°C erhöht. In der Flüssigkeit und im Dampfraum wurden Proben (Edelstahl V4A) der Werkstoff-Nr. 1.4571 angebracht. Nach Ende der Umsetzung gab man 96 %ige Schwefelsäure zu, bis pH 2.3 erreicht war und gab als fluoridabfangende Mittel 40 g hochdisperse Kieselsäure (Aerosil®) und 38.0 g Calciumhydroxid zu, wobei der pH-Wert sich auf 4.2 einstellte. Man begann, Wasserdampf einzuleiten, woraufhin mit dem Wasserdampf 5-Fluor-2-nitrophenol abdestillierte. Der pH-Wert wurde während der Wasserdampfdestillation mittels 96 %iger Schwefelsäure binnen 1.5 h auf 1.5 abgesenkt und danach bei diesem Wert gehalten. Das Destillat wurde auf 10°C gekühlt und danach abfiltriert. Man erhielt 91.9 g (0.58 Mol, 78 %) 5-Fluor-2-nitrophenol als gelben Festkörper.
Der Rührer erwies sich nach mehrfacher Wiederholung des Versuches als unverändert.
Proben der Werkstoffe 1.4462 und 1.4539 wiesen bereits nach 50 h Belastungszeit gleichmäßigen Angriff (integrale Abtragungsgeschwindigkeiten zwischen 1 und 6 mm/a) sowie teilweise Lochfraß- und Spannungsrißkorrosion auf.

### Beispiel 3

### Herstellung von 2,6-Difluorbenzoesäure aus 2,6-Difluorbenzonitril

In einem PTFE-Reaktor der in Beispiel 2 beschriebenen Konfiguration wurden bei 20°C 139 g (1 Mol) 2,6-Difluorbenzonitril in 235 g 75 %ige Schwefelsäure eingetragen und mit Stickstoff überlagert. Die Mischung wurde binnen 1 h auf 150°C geheizt und danach 6 h bei 150°C gehalten. Nach dieser Zeit wurde abgekühlt, auf 750 g Eiswasser gegossen und das ausgefallene Produkt durch Filtration und anschließende Wäsche mit Wasser (3 mal je 200 g) sowie anschließendes Trocknen isoliert. Man erhielt 137 g (86.7 %, 0.867 Mol) farblose 2,6-Difluorbenzoesäure als Pulver. Die Materialbeschaffenheit des Rührers wurde nach 10 Chargen überprüft und erwies sich als unverändert.

## Patentansprüche

1. Verfahren zur Herstellung fluorhaltiger Verbindungen durch Reaktion in einem mit einem Rührer versehenen Behälter, dadurch gekennzeichnet, daß man mit einem Rührer arbeitet, der ganz oder teilweise aus einem Kohlenstoffmaterial aufgebaut ist.

2. Verfahren nach Anspruch 1, dadurch gekennzeichnet, daß auch der Behälter aus einem Kohlenstoffmaterial aufgebaut ist oder mit diesem Material ausgekleidet ist.

3. Verfahren nach Anspruch 1 oder 2, dadurch gekennzeichnet, daß das Kohlenstoffmaterial korrosionsbeständiger Elektrographit, mit Kohlenstoffasern verstärkter Kunststoff oder mit Kohlenstoffasern verstärkter Kohlenstoff ist.

4. Verfahren nach Anspruch 1 bis 3, dadurch gekennzeichnet, daß man Rührer und Behälter durch mit Epoxid-, Melamin-, Furan-, Alkyd-, Vinyl-, Polyester-, Urethan- oder Phenolharze gefüllte Materialien abdichtet.

5. Verfahren nach Anspruch 1 bis 3, dadurch gekennzeichnet, daß Dichtstellen bei Rührern und Behältern durch Schichten aus inertem Material, insbesondere durch Edelmetalle geschützt werden.

6. Verfahren nach Anspruch 1 bis 5, dadurch gekennzeichnet, daß man bei Temperaturen von -20° bis 220°C, insbesondere bei 50 bis 180°C arbeitet.

7. Verfahren nach Anspruch 1 bis 5, dadurch gekennzeichnet, daß man bei 0,05 bis 4 MPa, vorzugsweise bei 0,1 bis 2 MPa arbeitet.

8. Verfahren nach Anspruch 1 bis 7, dadurch gekennzeichnet, daß man fluorierte aliphatische oder aromatische Carbonsäuren oder Hydroxyverbindungen herstellt.

9. Verfahren nach Anspruch 1 bis 8, dadurch gekennzeichnet, daß man bei einem pH-Wert unter 7 in Anwesenheit von mindestens 1 ppm Fluorid-Ionen und gegebenenfalls weiteren Halogenid-Ionen arbeitet.
